# EUROPÄISCHE PATENTANMELDUNG

(11) **EP 0 913 482 A1**
(43) Veröffentlichungstag der Anmeldung: **06.05.1999**
(21) Anmeldenummer: 97118756.2
(22) Anmeldetag: 29.10.1997
(51) Int. Cl.: C12Q 1/02, C12N 15/62

(54) **Verfahren zur Bestimmung von Wirksubstanzen**

(71) Anmelder: Evotec BioSystems AG, 22525 Hamburg (DE)
(72) Erfinder: Pohlner, Johannes, Dr, 22415 Hamburg (DE); Strauss, Andreas, Waldhäuser Strasse 70/8, 72076 Tübingen (DE); Thumm, Günther, Waldhäuser Strasse 70/8, 72076 Tübingen (DE); Götz, Friedrich, Prof. Dr., Waldhäuser Strasse 70/8, 72076 Tübingen (DE)
(74) Vertreter: Meyers, Hans-Wilhelm, Dr.Dipl.-Chem.

(57) **Zusammenfassung**

Verfahren zur Bestimmung von Wirksubstanzen, welche die kovalente Verknüpfung von Polypeptiden an die Oberfläche von Gram-positiven Bakterien beeinflussen, mit folgenden Schritten:
a) Bereitstellen einer Probe Gram-positiver Bakterien, welche mindestens eine kovalent mit der Oberfläche der Gram-positiven Bakterien verbundene Reportersubstanz mit mindestens einer Eigenschaft tragen, wobei die mindestens eine Reportersubstanz ohne kovalente Bindung an die Oberfläche der Gram-positiven Bakterien eine bestimmbare Veränderung in mindestens einer ihrer Eigenschaften aufweist,
b) Inkontaktbringen der Probe mit einer möglichen Wirksubstanz, und
c) Bestimmen der mindestens einen Eigenschaft der Reportersubstanz der Gram-positiven Bakterien der Probe

SEQUENZPROTOKOLL

(1) ALLGEMEINE ANGABEN:

(i) ANMELDER:
(A) NAME: Evotec BioSystems GmbH
(B) STRASSE: Grandweg 64
(C) ORT: Hamburg
(E) LAND: Deutschland
(F) POSTLEITZAHL: D-22529

(ii) BEZEICHNUNG DER ERFINDUNG: Verfahren zum Identifizieren
einer Nukleinsaeure

(iii) ANZAHL DER SEQUENZEN: 5

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0,
Version #1.30 (EPA)

(2) ANGABEN ZU SEQ ID NO: 1:

(i) SEQUENZKENNZEICHEN:
(A) LÄNGE: 5 Aminosäuren
(B) ART: Aminosäure
(C) STRANGFORM: nicht bekannt
(D) TOPOLOGIE: nicht bekannt

(ii) ART DES MOLEKÜLS: Peptid

(iii) HYPOTHETISCH: NEIN

(xi) SEQUENZBESCHREIBUNG: SEQ ID NO: 1:

Leu Pro Xaa Thr Gly
1               5

(2) ANGABEN ZU SEQ ID NO: 2:

(i) SEQUENZKENNZEICHEN:
(A) LÄNGE: 35 Basenpaare
(B) ART: Nucleotid
(C) STRANGFORM: nicht bekannt
(D) TOPOLOGIE: linear

(ii) ART DES MOLEKÜLS: Sonstige Nucleinsäure

(iii) HYPOTHETISCH: NEIN

(xi) SEQUENZBESCHREIBUNG: SEQ ID NO: 2:

ATAAGGCGCC TTAGTTTAAT TATGCTTTGT GATTC
35

(2) ANGABEN ZU SEQ ID NO: 3:

(i) SEQUENZKENNZEICHEN:
(A) LÄNGE: 56 Basenpaare
(B) ART: Nucleotid
(C) STRANGFORM: nicht bekannt
(D) TOPOLOGIE: linear

(ii) ART DES MOLEKÜLS: Sonstige Nucleinsäure

(iii) HYPOTHETISCH: NEIN

(xi) SEQUENZBESCHREIBUNG: SEQ ID NO: 3:

CGCAGGAAGC TTACCACAAT CTAAGAAATC TGAAATATCT CAAGCAAGTG GAGAAG
56

(2) ANGABEN ZU SEQ ID NO: 4:

(i) SEQUENZKENNZEICHEN:
(A) LÄNGE: 38 Basenpaare
(B) ART: Nucleotid
(C) STRANGFORM: nicht bekannt
(D) TOPOLOGIE: linear

(ii) ART DES MOLEKÜLS: Sonstige Nucleinsäure

(iii) HYPOTHETISCH: NEIN

(xi) SEQUENZBESCHREIBUNG: SEQ ID NO: 4:

AATAAGGCGC CTCATTATCC ACCTGTTTCA GGTAGTTC
38

(2) ANGABEN ZU SEQ ID NO: 5:

(i) SEQUENZKENNZEICHEN:
(A) LÄNGE: 33 Basenpaare
(B) ART: Nucleotid
(C) STRANGFORM: nicht bekannt
(D) TOPOLOGIE: linear

(ii) ART DES MOLEKÜLS: Sonstige Nucleinsäure

(iii) HYPOTHETISCH: NEIN

(xi) SEQUENZBESCHREIBUNG: SEQ ID NO: 5:

ACGAAAGCTT ACCACAATCT AAGAAATCTG AAC
33

## Beschreibung

Gegenstand der vorliegenden Erfindung ist ein Verfahren zur Bestimmung von Wirksubstanzen, welche die kovalente Verknüpfung von Polypeptiden an die Oberfläche von Gram-positiven Bakterien beeinflussen, ein Verfahren zum Identifizieren einer. Nukleinsäure, die für einen Polypeptidfaktor kodiert, welcher die kovalente Verknüpfung von Polypeptiden an die Oberfläche von Grampositiven Bakterien beeinflußt sowie die nach diesem Verfahren erhältlichen Nukleinsäuren.

Angesichts des vermehrten Auftretens antibiotikaresistenter Stämme stellen durch Gram-positive Bakterien hervorgerufene Infektionen beim Menschen eine zunehmende therapeutische Herausforderung dar. Eine Vielzahl von bakteriellen Oberflächenproteinen ist mit der Pathogenese dieser Organismen verbunden. So sind zellwandverankerte Pathogenitätsfaktoren bekannt, welche die bakterielle Adhäsion durch Bindung an extrazelluläre Matrixkomponenten der Wirtsgewebe, wie z.B. Kollagen, fördern. Andere Faktoren binden Serumkomponenten wie IgG und verbergen somit die authentische Bakterienoberfläche vor dem Immunsystem des Wirtes. Eine gezielte Hemmung der Verknüpfungsreaktion dieser Proteine an die bakterielle Zellwand ist daher von großem medizinischen Interesse.

Schneewind et al. (Cell, Vol. 70, p. 267 - 281, 1992) untersuchen den Verankerungsmechanismus von Protein A in der Zellwand von Staphylokokken. Protein A gehört zu einer wachsenden Klasse von Oberflächenproteinen Gram-positiver Bakterien, welche durch eine Abfolge des charakteristischen Sequenzmotives LPXTG, gefolgt von einer Gruppe aus 15 - 22 hydrophoben Aminosäuren sowie einer am C-terminalen Ende befindlichen Gruppe aus 5 - 12 geladenen Aminosäuren gekennzeichnet sind. Die Konservierung dieser Elemente wird als Indikation eines gemeinsamen Ausschleusungsmechanismus dieser Proteine in unterschiedlichen Grampositiven Spezies angesehen. Um die Lokalisation des Protein A (Unterscheidung zwischen in der Zellwand verankertem und sezerniertem Protein A) in *S. aureus* feststellen zu können, greifen die Autoren auf radioaktive Labellingverfahren zurück. Die Bedeutung der o.g. Sequenzelemente für die Zellwandverankerung wird mit Hilfe von Hypridproteinen sowie durch Mutagenese des LPXTG-Motives und des C-Terminus untermauert. Schneewind et al. befassen sich jedoch nicht mit möglicherweise die Verankerung der Oberflächenproteine katalysierenden Enzymen noch mit deren Hemmung.

Die Zellwandverankerungselemente in Oberflächenproteinen Gram-positiver Bakterien sind ebenfalls Thema eines weiteren Artikels von Schneewind et al. (EMBO J., Vol. 12, p. 4803 - 4811, 1993). Es wird gezeigt, daß Enterotoxin B, ein normalerweise in das Medium sezerniertes Protein, mittels C-terminaler Fusion an das Protein A-Verankerungssignal in der Staphylokokken-Zellwand verankert werden kann. Die Ergebnisse unterstützen die Hypothese, daß das Zellwand-Sorting mit einer proteolytischen Spaltung der Polypeptidkette am C-Terminus einhergeht. Vermutlich ist das LPXTG-Motiv die Stelle dieser Spaltung und kovalenter Zellwandverknüpfung, während der geladene Sequenzabschnitt als Retentionssignal während des Zellwand-Sortings dient. Die Relevanz der faltungsbedingten geometrischen Länge der hydrophoben Domäne wird experimentell bestätigt.

Mit dem Zelloberflächendisplay rekombinanter Proteine auf *Staphylococcus carnosus* befaßt sich ein Artikel von Samuelson et al. (J. Bacteriol., Vol. 177, no. 6, p. 1470 - 1476, 1995). Der Oberflächendisplay des Malariapeptides M3 erfolgt hier unter Verwendung von Promoter, Sekretionssignal und Propeptidregion des Lipasegens von *S. hyicus* sowie der Zellwandverankerungsregionen des Protein A von *S. aureus*.. Die hybride Proteinstruktur beinhaltet ferner ein Serumalbuminbindeprotein, welches der Detektion der rekombinanten, oberflächen-verankerten Proteine in einem colorimetrischen Sandwichassay dient. Weitere Detektionsmethoden umfassen die Immunogold-Elektronenmikroskopie, Immunofluoreszenzassay sowie das fluoreszenzaktivierte Cellsorting (FACS). Auch Samuelson et al. befassen sich nicht mit den genauen molekularen Mechanismen der Zellwandverankerung.

Die Struktur des Zellwandankers der Oberflächenproteine in *Staphylococcus aureus* ist Thema eines Reports von Schneewind et al. (Science, Vol. 268, p. 103 - 106, 1995). Die Autoren verwenden eine Kombination molekularbiologischer und massenspektrometrischer Techniken und können zeigen, daß nach Spaltung des Oberflächenproteins zwischen Threonin und Glycin des konservierten LPXTG-Motives die Carboxylgruppe des Threonins durch Transpeptidierung mit der freien Aminogruppe des Zellwand-Pentaglycins kovalent an den Mureinsacculus gebunden wird. Das für die Proteolyse und Transpeptidierung vermutlich verantwortliche Protein, die sogenannte Sortase, wird jedoch auch von Schneewind et al. nicht identifiziert bzw. charakterisiert.

Strauss und Götz (Molecular Microbiology, Vol. 21, p. 491 - 500, 1996) befassen sich mit der *in vivo* Immobilisierung enzymatisch aktiver Polypeptide auf der Zelloberfläche von *Staphylococcus carnosus*. Sie konstruieren ein Hybridprotein, welches aus *Staphylococcus hyicus* Lipase und der C-terminalen Region des *Staphylococcus aureus* Fibronectin Bindeprotein B (FnBPB) besteht. Um die Zellwandassoziation der prolipase bzw. des pro-LipFnBPB-Hybrides zu untersuchen, verwenden die Autoren ein prolipasespezifisches Antiserum in einem Immunofluoreszenzassay sowie Immunoblotting. Weitere Untersuchungen belegen, daß für eine effiziente Faltung der Lipase in ihre aktive Konformation offenbar ein Abstand von ca. 90 Aminosäuren zwischen dem C-Terminus des Enzyms und dem Zellwand-Sortingsignal unerläßlich ist. Der Einfluß größerer Abstände wurde an Fusionen aus proLip und der C-terminalen Region von *S. aureus* Protein A (proLipSPA, Spacer mit 165 Aminosäuren) und *S. aureus* Fibronectin Bindeprotein A (proLipFnBPA, Spacer mit 223 Aminosäuren) untersucht. Zusätzliche Experimente wurden mit *E. coli* β-Lactamase als Reportermolekül durchgeführt.

Die internationale Patentanmeldung PCT/US 96/14154 (Internationale Veröffent-lichungsnummer WO 97/08553) beschreibt ein Verfahren zum stabilen, nicht-kovalenten Display von Proteinen, Peptiden und anderen Substanzen auf der Oberfläche von Gram-positiven Bakterien. Es wurden vergleichende Untersuchungen zwischen dem nicht-kovalenten sowie den oben näher beschriebenen kovalenten Displayverfahren durchgeführt. Bei Ersatz des C-terminalen Sorting-Signals von Protein A, welches ein kovalentes Display generiert, durch das Zellwand Targeting-Signal von Lysostaphin (SPA_{CWT}) konnte eine im wesentlichen unveränderte Bindungsintensität von FITC-markiertem IgG an die Staphylokokken-Oberfläche beobachtet werden.

Das US-Patent 5,616,686 offenbart ein aus ca. 6 bis 20 Aminosäuren bestehendes Polypeptid, welches als integralen Bestandteil ein für die Verankerung von virulenzdeterminierenden Proteinen auf der Oberfläche von Gram-positiven Bakterien verantwortliches Peptidkonstrukt enthält. Insbesondere ist dieses Konstrukt dadurch gekennzeichnet, daß es an den Positionen 1, 2, 4 und 5 der Aminosäuresequenz die Aminosäuren L, P, T und G aufweist. Aufgrund der Homologie dieser Peptide mit den Sequenzen der Virulenzdeterminanten in den Wildtyp-Oberflächenproteinen reagieren erstere vermutlich mit den an der Verankerung beteiligten Enzyme. Hieraus resultiert, daß die Virulenzdeterminanten der Bakterien nicht oder nur in geringerem Maße verankert werden können und so ein Fortschreiten der Infektion unterbunden wird. Das bzw. die an der Oberflächen-verankerung beteiligte(n) Enzym bzw. Enzyme werden jedoch auch in dieser Patentschrift nicht charakterisiert.

Die internationale Patentanmeldung PCT/US 93/02355 (internationale Veröffent-lichungsnummer WO 93/18163) befaßt sich mit der Bereitstellung von Fusions-proteinen, welche mindestens die Ankerregion Gram-positiver Oberflächenproteine sowie variable Proteine, Polypeptide oder Peptide mit insbesondere therapeutischer Wirkung in Mensch und Tier enthalten. Die Ankerregion umfaßt ein LPSTGE-Segment, ein Spacer-Segment der Sequenz TAN, ein aus 20 Aminosäuren bestehendes hydrophobes Segment sowie einen geladenen Segmentabschnitt mit der Sequenz KRKEEN.

Es ist daher von Interesse, ein Verfahren bereitzustellen, welches die Identifizierung von Nukleinsäuren in Gram-positiven Bakterien erlaubt, die für solche Polypeptidfaktoren kodieren, die direkt oder indirekt die kovalente Verknüpfung von Polypeptiden an die Oberfläche der Gram-positiven Bakterien beeinflussen. Weiterhin ist es wünschenswert, ein Verfahren bereitzustellen, welches die Bestimmung von Wirksubstanzen erlaubt, die die kovalente Verknüpfung von Polypeptiden an die Oberfläche von Gram-positiven Bakterien direkt oder indirekt beeinflussen.

Überraschenderweise werden diese Ziele durch die Verfahren der vorliegenden Erfindung erreicht.

Polypeptide im Sinne der Erfindung bezeichnet aus in der Regel mindestens 20 Aminosäuren aufgebaute Polymere und umfaßt insbesondere auch Proteine. Die Aminosäuren sind durch den 1-Buchstaben-Code dargestellt, wobei X für eine beliebige Aminosäure steht.

Zunächst sollen im folgenden gemeinsame Grundlagen einer bevorzugten Ausführungsform der erfindungsgemäßen Verfahren dargestellt werden, bevor im einzelnen auf das Verfahren zur Identifizierung von Wirksubstanzen bzw. von Nukleinsäuren eingegangen wird.

Die erfindungsgemäßen Verfahren sind in einer bevorzugten Ausführungsform als enzymatische Reporterassays aufzufassen, die die Wirkung von Substanzen bzw. auch die Auswirkung von Mutationen auf bakterielle Faktoren (Targets) erfassen, welche direkt oder indirekt an der von einem LPXTG-Motiv abhängigen C-terminalen Verankerung von Polypeptiden an der Oberfläche von Gram-positiven Bakterien mitwirken. Aus der Vielzahl von Faktoren und Vorgängen, die sich auf diesen Prozess auswirken können, zielen die vorliegenden Verfahren vorzugsweise auf solche enzymatische Schritte, die nach dem Beginn der Translokation der Zelloberflächen-Polypeptide über die Cytoplasmamembran stattfinden. Darüberhinaus erfassen die erfindungsgemäßen Verfahren zum Teil enzymatische und andere Targets, die an der Biosynthese des Zellwandmureins mitwirken. Anhand der phänotypischen Merkmale der im jeweiligen Verfahren verwendeten Zellen lassen sich potentielle Wirkstoffe bzw. Mutationen bestimmten Targetgruppen zuordnen.

In einer besonders bevorzugten Ausführungsform der erfindungsgemäßen Verfahren bildet die zelluläre und molekulare Grundlage der. Reporterassays ein rekombinanter *Staphylococcus carnosus* Klon, welcher ein selektierbares Expressionsplasmid mit einer induzierbaren Reportergenfusion enthält. Die Genfusion kodiert für ein Hybridpolypeptid bestehend aus einem N-terminalen Signalpeptid, einem Vorläuferprotein der Lipase aus *Staphylococcus hyicus* und einem C-terminalen Anteil des Fibronektin-Bindeproteins B (FnBpB) aus *Staphylococcus aureus*. Nach seiner Produktion im Cytoplasma wird das Hybridpolypeptid aufgrund seiner N-terminalen Signalstrukturen durch die bakterielle Zellmembran transportiert und am Aminoende durch eine Signalpeptidase prozessiert. Ferner erfolgt eine Spaltung im C-terminalen LPXTG-Erkennungsmotiv und das verbleibende Hybridprotein wird kovalent mit dem Murein verknüpft. Auf experimentellem Wege wurde ermittelt, daß unterschiedliche Längen des FnBPB-Anteils die Ausbildung enzymatischer Aktivität der Lipasefusionen in unterschiedlicher Weise beeinflussen. Ein Konstrukt wurde identifiziert, das in seiner Zelloberflächen-gebundenen Form keine Lipaseaktivität aufwies (kodiert von Plasmid pTX30Δ82). Wurde die entsprechende Fusion jedoch nach ihrer kovalenten Verankerung durch Lysostaphinbehandlung von der Bakterienoberfläche freigesetzt, wurde die volle Lipaseaktivität erzielt. Im Rahmen der vorliegenden Erfindung wurde erstmals erkannt, daß in dem betreffenden Assayklon Störungen in der Zellwandverankerung der Lipasefusion zur Freisetzung der Fusion verbunden mit dem Auftreten von Lipaseaktivität in den Kulturüberständen führen. Als mögliche Angriffspunkte kommen verschiedene zelluläre Faktoren in Betracht, die entsprechend ihrem Wachstums-verhalten im wesentlichen in zwei Gruppen eingeteilt werden können.

Ein Target bzw. eine Targetgruppe ist das als Sortase bezeichnete Enzym bzw. Enzymkomplex, das bzw. der an der Oberfläche der Bakterien die carboxyterminale Spaltung relevanter Polypeptide im LPXTG-Motiv und deren anschließende kovalente Verknüpfung an Peptidbestandteile des Zellwandmureins wie z.B. Interpeptidbrücken, insbesondere Pentaglycineinheiten, bewerkstelligt. Die Inhibierung dieser Funktionen führt zwar vermutlich zur Freisetzung oberflächengebundener Faktoren und damit vermutlich zur Attenuierung pathogener Bakterien, vermutlich aber nicht zu maßgeblichen Beeinträchtigungen in der Lebens- und Teilungsfähigkeit der Bakterien. Kennzeichnendes phänotypisches Merkmal dieser Targetgruppe im Assayverfahren ist die Freisetzung von Lipaseaktivität in das Assaymedium bei mehr oder weniger unbeeinträchtigtem Wachstumsverhalten der Bakterien. Im Gegensatz dazu führt die Beeinträchtigung eines Targets bzw. einer Targetgruppe mit essentieller Funktion in der Mureinsynthese zu massiven phänotypisch erfassbaren Veränderungen in der Lebens- und Teilungsfähigkeit der Bakterien.

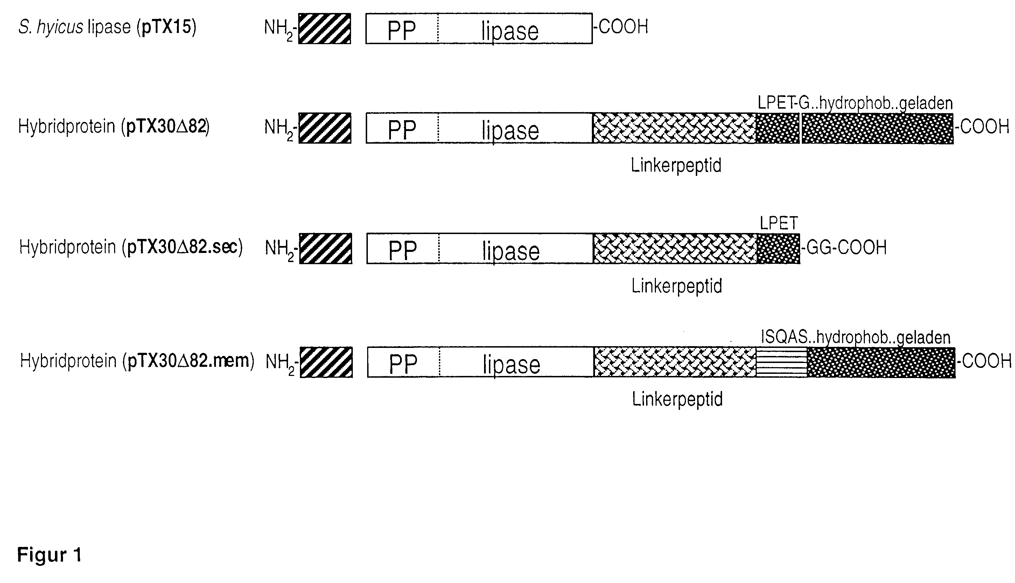

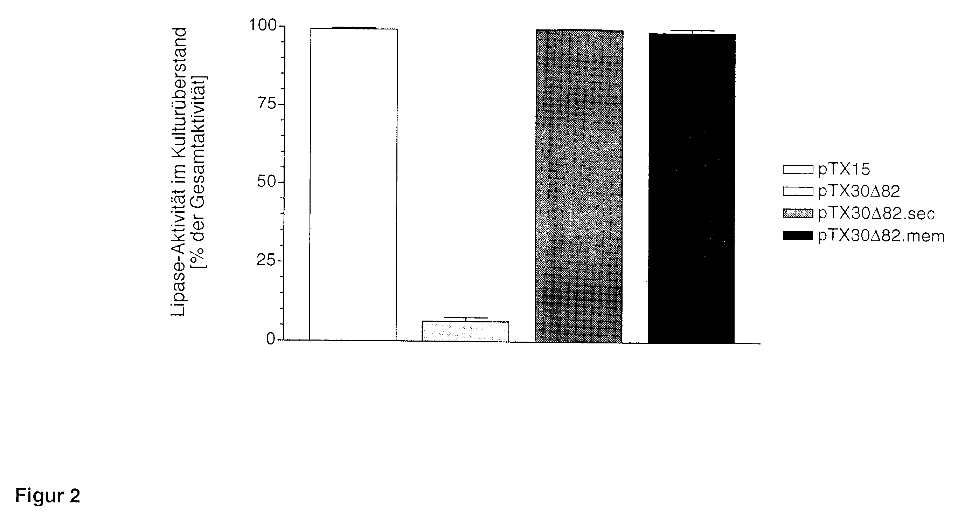

Um die potentielle Eignung der erfindungsgemäßen Verfahren für die Untersuchung des Targets "Sortase" zu demonstrieren, wurden zwei unterschiedliche Hemm-/Mutations-Szenarien mit Hilfe gentechnisch gezielt veränderter Hybridproteine simuliert (siehe hierzu Ausführungsbeispiel 1 sowie Figuren 1 und 2).

*S. carnosus*/ pTX30Δ82.mem produziert nach Induktion des Xylose-Promoters ein Hybridprotein bestehend aus der *S. hyicus* Prolipase und dem C-terminalen Fragment des *S. aureus* Fibronectin-Bindeprotein B (FnBPB), wobei das für die Verankerung mit der Zellwand wichtige LPXTG-Motiv gegen die Sequenz ISQAS ausgetauscht wurde (Figur 1). In anderen Ausführungsformen kann jedoch auch ein Austausch gegen eine beliebige andere, bevorzugt aus 5 Aminosäuren bestehende Sequenz erfolgen. In einer weiteren Ausführungsform ist für den mem-Phänotyp eine LPXTG-Substituion nicht notwendig, sondern auch eine komplette Deletion des LPXTG-Motivs denkbar. Damit entfällt die spezifische Spaltung am LPXTG-Motiv und somit die kovalente Verknüpfung an den Mureinsacculus. Überraschenderweise konnte gezeigt werden, daß die hydrophobe Ankersequenz, die somit am Carboxylende der Fusion erhalten bleibt, offenbar nicht ausreichend ist, um die Lipase stabil in der Zellhülle und somit an der Zelloberfläche zu verankern. Es kommt zur quantitativen Freisetzung der Lipaseaktivität von der Bakterienoberfläche in das Kulturmedium. Dieser Klon simuliert somit die Inhibition der Spaltungsreaktion durch die Sortase, die als wesentlicher Schritt der kovalenten Verknüpfung des N-terminalen Spaltprodukts an das Murein der Zellwand vorangeht.

*S. carnosus*/pTX30Δ82.sec produziert nach Induktion des Xylose-Promoters ein Hybridprotein bestehend aus der *S. hyicus* Prolipase und einem C-terminalen Fragment des *S. aureus* Fibronectin-Bindeprotein B (FnBPB), welches mit dem Motiv LPETGG endet (Figur 1). Dieser Klon simuliert die Inhibition der (kovalenten) Verknüpfungsreaktion zwischen dem zu verankernden, C-terminal bereits prozessierten Lipase-Hybridprotein und der Zellwand. Das Lipase-Hybridprotein wird von diesem Klon quantitativ in aktiver Konformation in den Kulturüberstand freigesetzt.

Die Bestimmung von Wirksubstanzen, welche die kovalente Verknüpfung von Polypeptiden an die Oberfläche von Gram-positiven Bakterien beeinflussen, erfolgt erfindungsgemäß durch ein Verfahren mit den folgenden Schritten:
a) Bereitstellen einer Probe Gram-positiver Bakterien, welche mindestens eine kovalent mit der Oberfläche der Gram-positiven Bakterien verbundene Reportersubstanz mit mindestens einer Eigenschaft tragen, wobei die mindestens eine Reportersubstanz ohne kovalente Bindung an die Oberfläche der Gram-positiven Bakterien eine bestimmbare Veränderung in mindestens einer ihrer Eigenschaften aufweist,
b) Inkontaktbringen der Probe mit einer möglichen Wirksubstanz,
c) Bestimmen der mindestens einen Eigenschaft der Reportersubstanz der Gram-positiven Bakterien der Probe.

Die Bestimmung der mindestens einen Eigenschaft der Reportersubstanz der Gram-positiven Bakterien der Probe kann in bevorzugter Weise im Vergleich zu mindestens einer Referenzprobe, die nicht mit der Wirksubstanz in Kontakt gebracht wurde, und/oder mindestens einer Referenzprobe, in der die Reportersubstanz ohne kovalente Bindung an die Oberfläche Gram-positiver Bakterien vorliegt, erfolgen.

Das erfindungsgemäße Verfahren erlaubt in vorteilhafter Weise selektiv die Identifizierung von solchen Wirksubstanzen, welche die kovalente Verknüpfung von Polypeptiden an die Oberfläche von Gram-positiven Bakterien direkt oder indirekt beeinflussen. Wie eingangs erläutert, umfaßt der putative Prozeß der Oberflächen-verankerung zwei spezifische Schritte:
a) Spaltung zwischen Threonin und Glycin des LPXTG-Motives und
b) kovalente Verknüpfung des Threonins an Peptidbestandteile, insbesondere an die Interpeptidbrücke, der Zellwand.

Mittels des erfindungsgemäßen Verfahrens werden sowohl solche Wirksubstanzen erfaßt, die die Spaltungsreaktion inhibieren und somit auch eine kovalente Verknüpfung unterbinden als auch solche Wirksubstanzen, die eine inhibitorische Funktion im zweiten Schritt der Oberflächenverankerung besitzen. Diese Szenarien wurden, wie eingangs dargestellt, mit Hilfe von gentechnisch gezielt veränderten Reportersubstanzen simuliert und somit die Grundlage für das erfindungsgemäße Verfahren geschaffen. Das erfindungsgemäße Verfahren erfaßt zudem auch solche Wirksubstanzen, die einen inhibierenden Einfluß auf die Zellwandbiosynthese haben, so daß als Folge eine kovalente Verknüpfung von Polypeptiden, insbesondere Pathogenitätsfaktoren, an die Zellwand, hier insbesondere an Interpeptidbrücken des Mureinsacculus, nicht oder nur noch eingeschränkt möglich ist. Darüberhinaus erfaßt es auch solche Substanzen, die zum aktiven Abbau der Zellwand führen.

In einer bevorzugten Ausführungsform des erfindungsgemäßen Verfahrens wird als Reportersubstanz ein Hybridpolypeptid verwendet, welches insbesondere eine Anordnung der folgenden Sequenzabschnitte aufweist: N-terminales Signalpeptid, Enzym, Sequenzabschnitt mit der Sequenz LPXTG, hydrophober Sequenzabschnitt und geladener Sequenzabschnitt. Das Signalpeptid wird im Rahmen des sekretorischen Prozessierungsweges proteolytisch entfernt. Wie in den nachfolgenden Beispielen gezeigt, kann insbesondere als enzymatischer Bestandteil des Hybridpolypeptides *S. hyicus* Lipase verwendet werden. Es kann jedoch auch von Vorteil sein, *E. coli* β-Lactamase oder andere Enzyme zu verwenden. Natürlich vorkommende Oberflächenpolypeptide können ebenso wie gentechnisch hergestellte Hybridpolypeptide als Reportersubstanzen dienen, die infolge der Wirkung geeigneter Wirksubstanzen im Medium mit Hilfe der gesamten Palette der bekannten chemischen, biochemischen und immunologischen Methoden nachgewiesen werden können. Um allerdings einen hohen Probendurchsatz bei einer ungeminderten Verläßlichkeit des erfindungsgemäßen Verfahrens zu gewährleisten, ist es insbesondere vorteilhaft, als Reportersubstanzen Hybridpolypeptide mit enzymatischer Aktivität zu verwenden. Wirksubstanzen, welche die proteolytische Spaltung des Signalpeptides wie auch den gesamten Transportweg durch die cytoplasmatische Membran beeinflussen, führen dagegen nicht zum Auftreten der aktiven Reportersubstanz im Überstand.

Es kann weiterhin bevorzugt sein, das Enzym als Proenzym bereitzustellen.

In einer bevorzugten Ausführungsform stellt die enzymatische Aktivität des Hybrid-polypeptides die mindestens eine Eigenschaft der Reportersubstanz dar. Es kann insbesondere bevorzugt sein, als bestimmbare Veränderung der mindestens einen Eigenschaft den Übergang des Enzyms aus einer inaktiven in eine aktive Konformation zu bestimmen. Dies kann bevorzugt durch Verwendung eines zwischen Enzym und LPXTG-Motiv angeordnetem Linkerpeptid erzielt werden. In einer bevorzugten Ausführungsform des erfindungsgemäßen Verfahrens ist die Anzahl der Aminosäuren des Linkerpeptides so gewählt, daß das Enzym in einer inaktiven Konformation an der Oberfläche der Gram-positiven Bakterien verankert wird. Bei Verwendung von *S. hyicus* Lipase sollte die Anzahl der Aminosäuren im Linkerpeptid insbesondere weniger als zehn betragen. In einer weiteren Ausführungsform könnte unter Verzicht auf das Linkerpeptid das Enzym mit seinem C-Terminus direkt an das LPXTG-Motiv fusioniert vorliegen. In Abwesenheit der Wirksubstanz tragen die Gram-positiven Bakterien somit kovalent an ihrer Oberfläche gebundene inaktive Enzyme, welche bei nicht-kovalenter Verknüpfung, insbesondere bei Freisetzung von der Oberfläche der Gram-positiven Bakterien, in eine aktive Konformation falten und somit eine bestimmbare Veränderung in einer ihrer Eigenschaften, d.h. in diesem Fall der enzymatischen Aktivität, erfahren.

Es ist insbesondere bevorzugt, das erfindungsgemäße Verfahren mit solchen Gram-positiven Bakterien durchzuführen, die einen geringen natürlichen Zellwand-turn-over und/oder eine geringe Anzahl an Zellwandproteasen und/oder eine geringe Anzahl an sekretierten Proteasen aufweisen, um den Anteil an falschpositiven Ergebnissen bei der Suche nach geeigneten Wirksubstanzen zu minimieren. Somit kann das erfindungsgemäße Verfahren nicht nur mit natürlich vorkommenden Gram-positiven Bakterien wie z.B. *S. carnosus* durchgeführt werden, sondern auch mit bereits genetisch veränderten Bakterien.

In einer besonders bevorzugten Ausführungsform des erfindungsgemäßen Verfahrens erfolgt die Bestimmung der mindestens einen Eigenschaft der Reportersubstanz mittels Fluoreszenzspektroskopie, insbesondere konfokaler Fluoreszenzspektroskopie wie in Ausführungsbeispiel 1 gezeigt. Es ist hierbei möglich, auf die bekannten Verfahren der Ein- oder Mehrphotonenanregung zurückzugreifen. Als vorteilhafte Bestimmungsmethode hat sich insbesondere das Verfahren der Fluoreszenzkorrelationsspektroskopie (FCS) erwiesen, wie es in der internationalen Patentanmeldung PCT/Ep 94/00117 (Internationale Veröffentlichungsnummer WO 94/16313) detailliert beschrieben ist. Anstelle der in der vorgenannten Patentanmeldung beschriebenen Vorrichtung kann es auch bevorzugt sein, FCS unter Verwendung von Elementen aus der Nahfeldspektroskopie durchzuführen, wie in dem deutschen Patent 44 38 391 und der korrespondierenden internationalen Patentanmeldung PCT/EP 95/04213 (internationale Veröffentlichungsnummer WO 96/13744) dargelegt.

Die europäische Patentanmeldung 96 116 373.0 beschreibt ein Verfahren zur Analyse von Partikel enthaltenden Proben durch wiederholte Messung der Photonenanzahl pro definiertem Zeitintervall von emittiertem oder in der Probe gestreutem Licht, gefolgt von der. Bestimmung der Verteilungsfunktion der Photonenanzahl pro definiertem Zeitintervall, aus welcher sodann die Verteilungsfunktion der Partikelhelligkeiten bestimmt wird. Dieses Verfahren kann in bevorzugter Weise auch zur Untersuchung lumineszenter, insbesondere fluores-zierender, Proben eingesetzt werden und wird in dieser spezifischen Ausführungsform als sogenannte Fluoreszenzintensitätsverteilungsanalyse (fluorescence intensity distribution analysis, FIDA) bezeichnet.

Für Fluoreszenzmessungen geeignete Farbstoffe sind dem Fachmann aus der Literatur bekannt. Es kann z.B. bevorzugt sein, die Umsetzung eines Substrates, welches eine Änderung in seinen Fluoreszenzeigenschaften erfährt, zu bestimmen. Weiterhin kann es bevorzugt sein, einen Reporterassay unter Verwendung von fluoreszierenden bzw. luminogenen Proteinen wie beispielsweise GFP (Green Fluorescent Protein) einzusetzen.

Die Erfindung betrifft ferner ein Verfahren zum Identifizieren einer Nukleinsäure, die für einen Polypeptidfaktor kodiert, welcher die kovalente Verknüpfung von Polypeptiden an die Oberfläche von Gram-positiven Bakterien beeinflusst, mit folgenden Schritten:
a) Bereitstellen einer Probe Gram-positiver Bakterien, welche genetisch veränderbar sind und mindestens eine kovalent mit der Oberfläche der Gram-positiven Bakterien verbundene Reportersubstanz mit mindestens einer Eigenschaft tragen, wobei die mindestens eine Reportersubstanz ohne kovalente Bindung an die Oberfläche eine bestimmbare Veränderung in mindestens einer ihrer Eigenschaften aufweist,
b) Verursachen von genetischen Veränderungen in Gram-positiven Bakterien der Probe,
c) Bestimmen der mindestens einen Eigenschaft der Reportersubstanz der Gram-positiven Bakterien der Probe,
d) Separieren von Gram-positiven Bakterien mit mindestens einer veränderten Eigenschaft der Reportersubstanz,
e) Isolieren der Nukleinsäure der in Schritt d) separierten Gram-positiven Bakterien,
f) Identifizieren mindestens eines Abschnittes der in Schritt
e) isolierten Nukleinsäure, der die genetische Veränderung trägt,
g) Identifizieren einer Nukleinsäure, die für einen Polypeptidfaktor kodiert, welcher die kovalente Verknüpfung von Polypeptiden an die Oberfläche von Gram-positiven Bakterien beeinflusst, auf der Grundlage des in Schritt f) identifizierten Abschnittes.

Auch in diesem Verfahren ist es bevorzugt, die mindestens eine Eigenschaft der Reportersubstanz im Vergleich zu mindestens einer Referenzprobe, die nicht genetisch verändert wurde, und/oder mindestens einer Referenzprobe, in der die Reportersubstanz ohne kovalente Bindung an die Oberfläche Gram-positiver Bakterien vorliegt, zu bestimmen.

Es kann bevorzugt sein, die gemäß Schritt g) des Verfahrens identifizierte Nukleinsäure zu gewinnen. Die von dieser Nukleinsäure kodierten Polypeptide können insbesondere im Wirkstoffscreening Verwendung finden.

Die kovalente Verknüpfung der Polypeptide erfolgt, wie bereits beschrieben, bevorzugt an das Murein der Zellwand, insbesondere an Interpeptidbrücken wie z.B. Pentaglycine der Zellwand Gram-positiver Bakterien. Bei den Polypeptiden handelt es sich insbesondere um Pathogenitätsfaktoren der Gram-positiven Bakterien.

Auch hier ist es bevorzugt, als Reportersubstanz ein Hybridpolypeptid zu verwenden, welches insbesondere die folgenden Sequenzabschnitte aufweist: N-terminales Signalpeptid, Enzym, Sequenzabschnitt mit der Sequenz LPXTG (Seq. ID No. 1), hydrophober Sequenzabschnitt und geladener Sequenzabschnitt. Wie in den nachfolgenden Beispielen gezeigt, kann insbesondere als enzymatischer Bestandteil des Hybridpolypeptides *S. hyicus* Lipase verwendet werden. Es kann jedoch auch von Vorteil sein, *E. coli* β-Lactamase zu verwenden. Natürlich vorkommende Oberflächenpolypeptide können ebenso wie gentechnische hergestellte Hybridpolypeptide als Reportersubstanzen dienen, die infolge der Wirkung geeigneter Wirksubstanzen im Medium mit Hilfe der gesamten Palette der bekannten chemischen, biochemischen und immunologischen Methoden nachgewiesen werden können. Um allerdings einen hohen Probendurchsatz bei einer ungeminderten Verläßlichkeit des erfindungsgemäßen Verfahrens zu gewährleisten, ist es insbesondere vorteilhaft, als Reportersubstanzen Hybridpolypeptide mit enzymatischer Aktivität zu verwenden.

Es kann weiterhin bevorzugt sein, das Enzym als Proenzym bereitzustellen.

In einer bevorzugten Ausführungsform stellt die enzymatische Aktivität des Hybridpolypeptides die mindestens eine Eigenschaft der Reportersubstanz dar. Es kann insbesondere bevorzugt sein, als bestimmbare Veränderung der mindestens einen Eigenschaft den Übergang des Enzyms aus einer inaktiven in eine aktive Konformation zu bestimmen. Dies kann bevorzugt durch Verwendung eines zwischen Enzym und LPXTG-Motiv angeordnetem Linkerpeptides erzielt werden. In einer bevorzugten Ausführungsform des erfindungsgemäßen Verfahrens ist die Anzahl der Aminosäuren des Linkerpeptides so gewählt, daß das Enzym in einer inaktiven Konformation an der Oberfläche der Gram-positiven Bakterien verankert wird. Bei Verwendung von *S. hyicus* Lipase sollte die Anzahl der Aminosäuren im Linkerpeptid insbesondere weniger als zehn betragen. In einer weiteren Ausführungsform könnte unter Verzicht auf das Linkerpeptid das Enzym mit seinem C-Terminus direkt an das LPXTG-Motiv fusioniert vorliegen. In Abwesenheit der Wirksubstanz tragen die Gram-positiven Bakterien somit kovalent an ihrer Oberfläche gebundene inaktive Enzyme, welche bei nicht-kovalenter Verknüpfung, insbesondere bei Freisetzung von der Oberfläche der Gram-positiven Bakterien, in eine aktive Konformation falten und somit eine bestimmbare Veränderung in einer ihrer Eigenschaften, d.h. in diesem Fall der enzymatischen Aktivität, erfahren.

Es ist insbesondere bevorzugt, das erfindungsgemäße Verfahren mit solchen Gram-positiven Bakterien durchzuführen, die einen geringen natürlichen Zellwand-turn-over und/oder eine geringe Anzahl an Zellwandproteasen und/oder eine geringe Anzahl an sekretierten Proteasen aufweisen, um den Anteil an falschpositiven Ergebnissen bei der Suche nach geeigneten Wirksubstanzen zu minimieren. Somit kann das erfindungsgemäße Verfahren nicht nur mit natürlich vorkommenden Gram-positiven Bakterien wie z.B. *S. carnosus* durchgeführt werden, sondern auch mit bereits genetisch veränderten Bakterien.

In einer besonders bevorzugten Ausführungsform des erfindungsgemäßen Verfahrens erfolgt die Bestimmung der mindestens einen Eigenschaft der Reportersubstanz mittels Fluoreszenzspektroskopie, insbesondere konfokaler Fluoreszenzspektroskopie wie in Ausführungsbeispiel 3 gezeigt. Es ist hierbei möglich, auf die bekannten Verfahren der Ein- oder Mehrphotonenanregung zurückzugreifen. Als vorteilhafte Bestimmungsmethode hat sich insbesondere das Verfahren der Fluoreszenzkorrelationsspektroskopie (FCS) erwiesen, wie es in der internationalen Patentanmeldung PCT/EP 94/00117 (Internationale Veröffentlichungsnummer WO 94/16313) detailliert beschrieben ist. Anstelle der in der vorgenannten Patentanmeldung beschriebenen Vorrichtung kann es auch bevorzugt sein, FCS unter Verwendung von Elementen aus der Nahfeldspektroskopie durchzuführen, wie in dem deutschen Patent 44 38 391 und der korrespondierenden internationalen Patentanmeldung PCT/EP 95/04213 (internationale Veröffentlichungsnummer WO 96/13744) dargelegt.

Die europäische Patentanmeldung 96 116 373.0 beschreibt ein Verfahren zur Analyse von Partikel enthaltenden Proben durch wiederholte Messung der Photonenanzahl pro definiertem Zeitintervall von emittiertem oder in der Probe gestreutem Licht, gefolgt von der Bestimmung der Verteilungsfunktion der Photonenanzahl pro definiertem Zeitintervall, aus welcher sodann die Verteilungsfunktion der Partikelhelligkeiten bestimmt wird. Dieses Verfahren kann in bevorzugter Weise auch zur Untersuchung lumineszenter, insbesondere fluoreszierender, Proben eingesetzt werden und wird in dieser spezifischen Ausführungsform als sogenannte Fluoreszenzintensitätsverteilungsanalyse (fluorescence intensity distribution analysis, FIDA) bezeichnet.

Für Fluoreszenzmessungen geeignete Farbstoffe sind dem Fachmann aus der Literatur bekannt. Es kann z.B. bevorzugt sein, die Umsetzung eines Substrates, welches eine Änderung in seinen Fluoreszenzeigenschaften erfährt, zu bestimmen. Weiterhin kann es bevorzugt sein, einen Reporterassay unter Verwendung von fluoreszierenden bzw. luminogenen Proteinen wie GFP (Green Fluorescent Protein) einzusetzen.

Die genetische Veränderung gemäß Schritt b) des erfindungsgemäßen Verfahrens erfolgt in einer bevorzugten Ausführungsform mittels Transposon-Mutagenese. Stattdessen kann jedoch auch z.B. eine UV-Mutagenese oder eine chemische Mutagenese durchgeführt werden. Derartige Verfahren sind in zahlreichen Publikationen oder auch in Standard-Werken beschrieben (Miller J.H. (Ed): Experiments in Molecular Genetics, Cold Spring Harbor, New York 11724, Cold Spring Harbor Laboratory, 1977; Sambrock J., Fritsch E.F. und Maniatis T. (Eds): Molecular cloning, a laboratory manual; New York, Cold Spring Harbor Laboratory, 1989).

Der beispielhaft gewählte, nicht-pathogene Organismus *S. carnosus* zeigt eine ausgesprochen geringe Freisetzung von Zellwand-Untereinheiten und produziert keine störenden Exo-Proteasen. Seine Verwandtschaft zu den pathogenen Staphylococcen, wie *S*. *aureus*, *S. hyicus* oder *S. epidermidis*, ermöglicht den Transfer der hier beispielhaft erzielten Ergebnisse auf medizinisch relevantere Organismen.

So ist es beispielsweise möglich, das erfindungsgemäße Verfahren hinsichtlich der Schritte a) bis f) in dem nicht-pathogenen Organismus *S. carnosus* durchzuführen. Der gemäß Schritt f) identifizierte Abschnitt kann dann als Grundlage dienen, um nicht nur im Wildtyp-Stamm, sondern auch in verwandten Stämmen solche Nukleinsäuren zu identifizieren, die für einen Polypeptidfaktor kodieren, welcher die kovalente Verknüpfung von Polypeptiden an die Oberfläche dieser Stämme beeinflusst. Dies kann in dem Fachmann bekannter Weise erfolgen, wie z.B. mittels Hybridisierung mit Gensonden oder mittels PCR-Amplifikation der entsprechenden Gene.

In einer weiteren Ausführungsformen des erfindungsgemäßen Verfahrens ist es ebenso möglich, Gruppen von möglichen Wirksubstanzen der Probe Gram-positiver Bakterien zuzugeben, um bei positivem Signal eine weitere Differenzierung dieser Klasse vorzunehmen.

Desweiteren kann der Probe, falls eine mögliche Wirksubstanz nicht positiv mit der Probe interferiert, eine weitere mögliche Wirksubstanz zugegeben werden, ohne die Probe zu wechseln. In diesem Fall werden sequentiell eine mögliche Wirksubstanz zugegeben. In diesem Falle ist es auch möglich, synergistische Effekte festzustellen, wenn sich die als wirksam erwiesene Substanz in einem Bestätigungsversuch anders verhält als im Gemisch mit der oder den scheinbar nicht wirksamen Substanzen.

Gegenstand der vorliegenden Erfindung sind ferner die mittels des erfindungsgemäßen Verfahrens identifizierten und bevorzugt isolierten Nukleinsäuren.

Die angegebenen spezifischen Nukleinsäuresequenzen wurden durch Sequenzierung der an die Insertionsstellen der Transposons angrenzenden Bereich der genomischen S. canosus DNA erhalten.

Die Figur 1 beschreibt beispielhaft die Struktur eines als Reportersubstanz einsetzbaren Hybridproteins (Assay-Plasmid pTX30Δ82) sowie die oben erwähnten Strukturen der zur Simulation der erfindungsgemäßen Verfahren verwendeten Hybridproteine (Plasmide pTX30Δ82.sec und pTX30Δ82.mem) im Vergleich zur Struktur der *S. hyicus* Lipase (Plasmid pTX15). Am N-Terminus befindet sich das sog. Signalpeptid (schraffiert dargestellt), welches den Transport des Proenzyms (schematisch durch PP und Lipase dargestellt) durch die cytoplasmatische Membran ermöglicht und im Rahmen des sekretorischen Prozessierungsprozesses proteolytisch entfernt wird. In bevorzugter Weise schließt sich an die Lipase ein Linkerpeptid an, dessen Länge derart gewählt ist, daß die Lipase in einer inaktiven Konformation an der Oberfläche der Gram-positiven Bakterien kovalent verankert werden kann. Es folgen LPETG-Motiv, hydrophober und geladener Sequenzabschnitt (Assay-Plasmid pTX30Δ82). Das LPXTG-Motiv ist im zur Simulation verwendeten Hybridprotein (Plasmid pTX30Δ82.mem) durch die Sequenzabfolge ISQAS ersetzt. In dem zu einer weiteren Simulation verwendeten Hybridprotein (Plasmid pTX30Δ82.sec) schließt sich an das Linkerpeptid lediglich ein Sequenzabschnitt mit der Sequenz LPETGG an.

### Ausführungsbeispiel 1

### Simulationsszenarien

### Stämme & Plasmide

Wildtyp-Stamm *S. carnosus* TM300 (Götz, F., J. Appl. Bacteriol. Symp. Supp. 69: 49-53, 1990) wurde als Wirtsorganismus für die Herstellung aller rekombinanter Staphylokokkenstämme benutzt. Die Herstellung des Assay-Plasmids pTX30Δ82 wird nachfolgend in Ausführungsbeispiel 2 beschrieben. Die Plasmide pTX30Δ82.mem und pTX30Δ82.sec wurden analog zum Plasmid pTX30Δ82 unter Verwendung der Oligonukleotidpaare 
(Seq. ID No. 2 bis 5) AS14(5'-ATAAGGCGCCTTAGTTTAATTATGCTTTGTGATTC),
/AS45(5´-CGCAGGAAGCTTACCACAATCTAAGAAATCTGAAATATCTCAAGCAAGTGGAG-
AAG) und AS42(5´-AATAAGGCGCCTCATTATCCACCTGTTTCAGGTAGTTC) /
AS22(5´-ACGAAAGCTTACCACAATCTAAGAAATCTGAAC)
 ausgehend von pTX30 hergestellt. Die Plasmide pTX30Δ82, pTX30Δ82.mem und pTX30Δ82.sec wurden in *S. carnosus* TM300 transformiert (Götz & Schumacher, FEMS Microbiol. Lett. 40: 285-288, 1987).

### Medien

Für die Kultivierung der Bakterien in Flüssigkultur wurde Basis-Medium (BM) verwendet, das 1% Pepton, 0.5% Hefe-Extrakt, 0.5% NaCl, 0.1% Glucose und 0.1% Di-Kaliumhydrogenphosphat enthielt (pH 7.4). Für die Induktion des Xylose Promoters wurde modifiziertes Basis-Medium (Induktionsmedium) benutzt, in dem die Glucose durch 0,5% Xylose ersetzt wurde. Nach Bedarf wurde das BM mit Chloramphenicol (Cm, 10 mg/l), Tetracyclin (Tc, 25 mg/l) und Erythromycin (Em, 2.5 mg/l) versetzt. Agar-Selektionsplatten enthielten zusätzlich 15 g/l Agar. Lipase-Testplatten wurden unter Verwendung von Tributyrin-Agar Basis (Merck) nach Anleitung des Herstellers zubereitet. Der Agar wurde vor dem Gießen der Platten mit 1% Glycerintributyrat und den entsprechenden Antibiotika (Tc, Em) versetzt. Auf diesen Platten können Lipasefreisetzende Bakterienzellen durch die Bildung klarer Höfe identifiziert werden.

### Part I:

### Lokalisation und Quantifizierung der Lipase-Aktivität in den Simulations-Klonen

Jeweils 5 ml Basismedium wurden von Platte mit Wildtyp-Stamm *S. carnosus* TM300 bzw. den oben beschriebenen *S. carnosus* Klonen beimpft und bei 37°C über Nacht unter Schütteln inkubiert. Mit diesen Vorkulturen wurden jeweils 5 ml Induktionsmedium 1:100 beimpft und bei 37°C bis zur spät-logarithmischen Wachstumsphase geschüttelt. Die Kulturen wurden auf 4°C gekühlt, bevor die Lipase-Aktivität im Kulturüberstand und die zellgebundene Lipase-Aktivität nach Freisetzung durch Lysostaphinbehandlung der Zellen gemäß Strauss und Götz (Mol. Microbiol. 21: 491-500, 1996) bestimmt wurde. Die Gesamtaktivität ergab sich aus der Summe der zellgebundenen Aktivität und der Aktivität im Kulturüberstand. Anschließend wurde die jeweilige Lipase-Aktivität im Kulturüberstand in Relation zur Gesamtaktivität des entsprechenden Klons bestimmt (Figur 2).

### Part II:

### Etablierung eines auf Fluoreszenzspektroskopie basierenden Assayverfahrens

Die *S. carnosus* Klone *S. carnosus*/pTX30Δ82, /pTX30Δ82.mem, und /pTX30Δ82 sec wurden über Nacht bei 37°C in Basis-Medium kultiviert. Die Zelldichten dieser Kulturen wurden durch Messung der optischen Dichten (OD576) im Photometer bestimmt und Verdünnungen (ca. 1:200) gleicher Zelldichte hergestellt. Für die, Herstellung dieser Verdünnungen wurde modifiziertes Basis-Medium (Induktionsmedium) verwendet. Die verdünnten Kulturen wurden daraufhin erneut bei 37°C angewachsen. In verschiedenen Experimenten wurden sowohl Mikrotiterplatten als auch andere Gefäße für die Kultivierungen verwendet.

Zu unterschiedlichen Zeitpunkten der Kultivierung wurde die von den Bakterien freigesetzte Lipaseaktivität in den Kulturüberständen bestimmt. Die Zellen wurden durch Zentrifugation sedimentiert, die Kulturüberstände abgehoben und, wenn notwendig, auf Eis aufbewahrt. Assays wurden in Mikrotiterplatten (100 µl) mit Glasboden durchgeführt Der Lipase-Assay-Puffer setzte sich wie folgt zusammen: 10 mM CaCl₂, 0,05% Triton-X-100, 20 mM Tris/HCl, pH 8,0. Als fluorogenes Farbsubstrat wurde 1,2-o-Dilauryl-rac-glycero-3-glutarsäure-resorufinester (Sigma # D7414) eingesetzt. Die Substrat-Stammlösung wurde in 100% DMSO bei 1 mg/ml angesetzt und bei -20°C gelagert. Pro Messprobe wurden jeweils 10 µl der Kulturüberstände mit 80 µl Lipase-Assay-Puffer und 10 µl Substrat-Lösung (10 µM Endkonzentration) vermischt. Die Umsetzung des Substrats wurde fluorometrisch unter Verwendung eines Fluoreszenz- (ELISA)Readers oder mittels Fluoreszenz-Korrelationsspektroskopie mit dem ConfoCor bestimmt.

Während der Klon *S. carnosus*/pTX30Δ82 in den Messungen nur unwesentlich höhere Signale als die Negativkontrolle (nur Puffer und Substrat) ergab, wurden in den Kulturüberständen der beiden Klone *S. carnosus*/pTX30Δ82.mem und *S. carnosus*/pTX30Δ82.sec signifikante Mengen an Lipaseaktivität gefunden.

### Ausführungsbeispiel 2

### Screening von Mutanten mit Defekten in der Oberflächenverankerung infolge einer Transposon-Mutagenese

### Stämme & Plasmide

Wildtyp-Stamm *S. carnosus* TM300 (Götz, F., J. Appl. Bacteriol. Symp. Supp. 69: 49-53, 1990) wurde wie von Götz und Schumacher (FEMS Microbiol. Lett. 40: 285-288, 1987) beschrieben mit dem Assay-Plasmid pTX30Δ82 transformiert. Plasmid pTX30Δ82 wurde analog zum Plasmid pCX30Δ82 (Strauss und Götz, Mol. Microbiol. 21:491-500, 1996) hergestellt. Es diente jedoch nicht das mit Chloramphenicol selektionierbare Plasmid pCX15 (Wieland et al., Gene 158: 91-96, 1995), sondern das mit Tetracyclin selektionierbare Plasmid pTX15 (Peschel et al., FEMS Microbiol. Lett. 137: 279-284, 1996) als Ausgangsvektor. Dieses Plasmid enthält eine Genfusion, welche das Assay-Hybridprotein bestehend aus *S. hyicus* Lipase und dem C-terminalen Teil des Fibronectin Bindeprotein B (FnBPB) kodiert und welche unter der Kontrolle des induzierbaren Xylose-Promotors steht (Wieland et al., Gene 158: 91-96, 1995). Der Abstand zwischen dem C-terminalen Alanin-Rest der Lipase und dem Leucin-Rest des LPXTG-Motives des FnBPB betrug 10 Aminosäuren (Figur 1). Für die Transposon-Mutagenese wurde dieser Stamm zusätzlich mit dem Plasmid pTV1ts (Youngman et al. in: Smith et al. (ed): Regulation of procaryotic development. American Society for Microbiology, Washington DC, 65-87, 1989) transformiert.

### Medien

Es wurden Medien entsprechend der Angaben in Ausführungsbeispiel 1 verwendet.

### Transposon-Mutagenese

Das Plasmid pTV1ts, welches in Gram-positiven Bakterien eine temperatur-sensitive Replikation zeigt, kodiert für eine Chloramphenicol-Resistenz und trägt das Transposon Tn917, das ein Erythromycin-Resistenzgen beinhaltet (Youngman et al. in: Smith et al. (ed): Regulation of procaryotic development. American Society for Microbiology, Washington DC, 65-87, 1989). *S*. *carnosus* Zellen, welche die Plasmide pTV1ts und pTX30Δ82 trugen, wurden über Nacht in BM-Selektions-medium (Cm, Tc, Em) bei 30 °C angezogen. Mit dieser Vorkultur wurde BM-Selektionsmedium (Tc, Em) 1:100 inocculiert, und das Tn917 bei der nicht-permissiven Temperatur von 40°C für mindestens 20 Zellgenerationen mobilisiert.

### Screening von Mutanten mit Defekten in der Oberflächenverankerung

Mutanten mit einer Transposon-Mobilisierungsrate (Zellen Tc+Em resistent, aber Cm sensitiv) von mindestens 1000:1 wurden direkt auf Lipase-Testplatten ausplattiert und bei 30°C für ca. 48 h bebrütet, bevor potentielle Mutanten mit Defekten in der kovalenten Zellwand-Verankerung identifiziert wurden. Diese zeichneten sich durch einen durch die freigesetzte Lipase-Aktivität erzeugten klaren Hof aus. Auf Agar-Selektionsplatten (Tc, Em) wurden die relevanten Klone nochmals vereinzelt, bevor ihr Phänotyp ein weiteres Mal auf Lipase-Testplatten und in Flüssigkultur verifiziert wurde. Hierzu wurden jeweils 5 ml Induktions-Medium von Platte beimpft und bei 30°C über Nacht geschüttelt, bevor die Lipaseaktivität im Kulturüberstand im Vergleich zum nicht-mutierten Ausgangsstamm, wie von Strauss und Götz (Mol. Microbiol. 21: 491-500, 1996) beschrieben, bestimmt wurde.

## Patentansprüche

1. Verfahren zur Bestimmung von Wirksubstanzen, welche die kovalente Verknüpfung von Polypeptiden an die Oberfläche von Gram-positiven Bakterien beeinflussen, mit folgenden Schritten:
a) Bereitstellen einer Probe Gram-positiver Bakterien, welche mindestens eine kovalent mit der Oberfläche der Gram-positiven Bakterien verbundene Reportersubstanz mit mindestens einer Eigenschaft tragen, wobei die mindestens eine Reportersubstanz ohne kovalente Bindung an die Oberfläche der Gram-positiven Bakterien eine bestimmbare Veränderung in mindestens einer ihrer Eigenschaften aufweist,
b) Inkontaktbringen der Probe mit einer möglichen Wirksubstanz, und
c) Bestimmen der mindestens einen Eigenschaft der Reportersubstanz der Gram-positiven Bakterien der Probe.

2. Verfahren nach Anspruch 1, dadurch gekennzeichnet, daß die mindestens eine Eigenschaft der Reportersubstanz im Vergleich zu mindestens einer Referenzprobe, die nicht mit der Wirksubstanz in Kontakt gebracht wurde, und/oder mindestens einer Referenzprobe, in der die Reportersubstanz ohne kovalente Bindung an die Oberfläche Gram-positiver Bakterien vorliegt, bestimmt wird.

3. Verfahren nach Ansprüchen 1 und/oder 2, dadurch gekennzeichnet, daß die kovalente Verknüpfung der Polypeptide an das Murein der Zellwand, insbesondere an Interpeptidbrücken wie z.B. Pentaglycine, Gram-positiver Bakterien erfolgt.

4. Verfahren nach mindestens einem der Ansprüche 1 bis 3, dadurch gekennzeichnet, daß die Polypeptide Pathogenitätsfaktoren Gram-positiver Bakterien darstellen.

5. Verfahren nach mindestens einem der Ansprüche 1 bis 4, dadurch gekennzeichnet, daß die Reportersubstanz ein Hybridpolypeptid ist.

6. Verfahren nach Anspruch 5, dadurch gekennzeichnet, daß das Hybridpolypeptid eine Anordnung der folgenden Sequenzabschnitte aufweist: N-terminales Signalpeptid, Enzym, Sequenzabschnitt mit der Sequenz LPXTG, hydrophober Sequenzabschnitt und geladener Sequenzabschnitt.

7. Verfahren nach Anspruch 6, dadurch gekennzeichnet, daß das Enzym als Proenzym bereitgestellt wird.

8. Verfahren nach Ansprüchen 6 und/oder 7, dadurch gekennzeichnet, daß die enzymatische Aktivität des Hybridpolypeptides die mindestens eine Eigenschaft der Reportersubstanz ist.

9. Verfahren nach mindestens einem der Ansprüche 6 bis 8, dadurch gekennzeichnet, daß die bestimmbare Veränderung der Übergang aus einer inaktiven in eine aktive Konformation des Enzyms ist.

10. Verfahren nach mindestens einem der Ansprüche 6 bis 9, dadurch gekennzeichnet, daß zwischen Enzym und Sequenzabschnitt mit der Sequenz LPXTG ein Linkerpeptid, welches insbesondere weniger als 10 Aminosäuren umfaßt, angeordnet ist.

11. Verfahren nach mindestens einem der Ansprüche 1 bis 10, dadurch gekennzeichnet, daß die Gram-positiven Bakterien einen geringen natürlichen Zellwand-turn-over und/oder eine geringe Anzahl an Zellwandproteasen und/oder eine geringe Anzahl an sekretierten Proteasen aufweisen.

12. Verfahren nach mindestens einem der Ansprüche 1 bis 11, dadurch gekennzeichnet, daß die Bestimmung der mindestens einen Eigenschaft der Reportersubstanz mittels Fluoreszenzspektroskopie, insbesondere konfokaler Fluoreszenzspektroskopie, erfolgt.
